# EUROPÄISCHE PATENTSCHRIFT

(11) **EP 0 940 381 B1**
(45) Veröffentlichungstag und Bekanntmachung des Hinweises auf die Patenterteilung: **05.11.2003**
(21) Anmeldenummer: 99103516.3
(22) Anmeldetag: 24.02.1999
(51) Int. Cl.: C07C 7/00, C07C 7/04, C07C 5/03, C10G 45/32, C10G 65/06

(54) **Verfahren zur Gewinnung von Cyclopentan und/oder Cyclopenten**
Process for the recovery of cyclopentane and/or cyclopentene
Procédé de récupération de cyclopentane et/ou de cyclopentène

(30) Priorität: 05.03.1998 DE 19809361; 27.10.1998 DE 19849425
(43) Veröffentlichungstag der Anmeldung: 08.09.1999
(73) Patentinhaber: BP Köln GmbH, D-50769 Köln (DE)
(72) Erfinder: Stüwe, Arnd, 51373 Leverkusen (DE); Wurz, Rolf Dieter, verstorben, (DE); Müller, Michael, Dr., 50769 Köln (DE); Herwig, Jens, Dr., 50859 Köln (DE); Gabel, Christian, Dr., 41539 Dormagen (DE); Grub, Joachim, Dr., 41539 Dormagen (DE)
(74) Vertreter: Weber, Thomas, Dr.Dipl.-Chem.

(56) Entgegenhaltungen:
- EP-A- 0 799 881
- DE-A- 1 643 947
- DE-A- 2 256 384

## Beschreibung

Die vorliegende Erfindung betrifft ein Verfahren zur Gewinnung von Cyclopentan aus einem partiell hydrierten Benzol-Vorlauf oder einer partiell hydrierten C₅-Fraktion durch destillative Auftrennung. In einer Variante des erfindungsgemäßen Verfahrens kann neben Cyclopentan zusätzlich Isopentan (2-Methylbutan) gewonnen werden.

Cyclopentan hat in jüngerer Zeit Bedeutung als Ersatzstoff für Fluor-Chlor-Kohlenwasserstoffe (FCKW) als Treibmittel für Polyurethan-Systeme gefunden, da es bezüglich der Schädigung der Ozonschicht der Erdatmosphäre als unschädlich anzusehen ist. Cyclopentan hat darüber hinaus Bedeutung als spezielles Lösungsmittel. Cyclopenten ist ein wertvolles Comonomer für Polymerisate und kann außerdem durch Hydrierung weiteres Cyclopentan liefern. Isopentan schließlich ist ein wertvoller Grundstoff für Alkylate und andere chemische Umsetzungen.

Beim Cracken von Naphtha (Chemiebenzin) in Steam-Crackern oder in Fluid Catalytic Crackem (FCC) entstehen neben Restgas (hauptsächlich H₂ und CH₄) so wertvolle Monomere wie Ethylen, Propen, Buten-(1), Buten-(2) und Isobuten und ferner Butadien (C₂-, C₃- und C₄-Fraktionen).

Der übrig bleibende Anteil an Cracker-Produkten, Pyrolysebenzin genannt, wird zur Gewinnung von Aromaten aufgearbeitet. Zunächst wird das Pyrolysebenzin partiell hydriert, um Acetylen-Verbindungen und Diolefine zu Alkenen bzw. Monoolefinen zu hydrieren. Anschließend wird aus dem partiell hydrierten Pyrolysebenzin durch Destillation ein partiell hydrierter Benzol-Vorlauf gewonnen, der eine'große Anzahl von isomeren C₅-Alkanen und C₅-Alkenen, daneben Cyclopentan und Cyclopenten, . geringe Mengen nicht vollständig abgetrennter C₄-Kohlenwasserstoffe sowie einen gewissen Anteil an Kohlenwasserstoffen mit 6 und mehr C-Atomen enthält. Alternativ dazu kann destillativ aus dem partiell hydrierten Pyrolysebenzin eine partiell hydrierte C₅-Fraktion gewonnen werden, die eine große Zahl isomerer C₅-Alkane und C₅-Alkene, Cyclopentan, Cyclopenten, kleine Mengen nicht vollständig abgetrennter C₄-Kohlenwasserstoffe sowie geringe Mengen an Kohlenwasserstoffen mit 6 und mehr C-Atomen enthält.

Die Aufarbeitung des Pyrolysebenzins kann auch so vorgenommen werden, daß zunächst durch destillative Trennung ein nicht hydrierter Benzol-Vorlauf anfällt. Dieser wird anschließend partiell hydriert, um wiederum Acetylen-Verbindungen und Diolefine zu Alkenen bzw. Monoolefinen zu hydrieren. Danach enthält dieser partiell hydrierte Benzol-Vorlauf eine große Anzahl von isomeren C₅-Alkanen und C₅-Alkenen, daneben Cyclopentan und Cyclopenten, geringe Mengen nicht vollständig abgetrennter C₄-Kohlenwasserstoffe sowie einen gewissen Anteil an Kohlenwasserstoffen mit 6 und mehr C-Atomen. Alternativ dazu kann die Aufarbeitung des Pyrolysebenzins auch so erfolgen, daß man zunächst nur C₅-Kohlenwasserstoffe destillativ abtrennt, wodurch eine nicht-hydrierte. C₅-Fraktion erhalten wird. Diese C₅-Fraktion wird anschließend ebenfalls hydriert und enthält danach eine große Zahl isomerer C₅-Alkane und C₅-Alkene, Cyclopentan, Cyclopenten, kleine Mengen nicht vollständig abgetrennter C₄-Kohlenwasserstoffe sowie geringere Mengen an Kohlenwasserstoffen mit 6 und mehr C-Atomen.

Eine typische Zusammensetzung eines partiell hydrierten Benzol-Vorlaufs oder einer partiell hydrierten C₅-Frakion, die nach einer der zuvor beschriebenen Verfahrensvarianten gewonnen wurden, ist die folgende, wobei die Summe der Gew.-% 100 ergibt.

| Komponente (Kohlenwasserstoffe = KW) | Gehalt (Gew.-%) |
|---|---|
| C₄-KW | 0 - 4 |
| Leichtsiedende C₅-KW | 30 - 60 |
| 2-Methylbuten-2 | 8 - 20 |
| Cyclopentan | 6 - 24 |
| Cyclopenten | 8 - 27 |
| C₆⁺-KW | 0 - 25 |
| C₅-Diene | 0-0,5 |

Die bevorzugte Zusammensetzung eines partiell hydrierten Benzol-Vorlaufs ist die folgende, wobei die Summe der Gew.-% 100 ergibt.

| Komponente (Kohlenwasserstoffe = KW) | Gehalt (Gew.-%) |
|---|---|
| C₄-KW | 0 - 3 |
| Leichtsiedende C₅-KW | 30 - 45 |
| 2-Methylbuten-2 | 8 - 15 |
| Cyclopentan | 6 - 18 |
| Cyclopenten | 8 - 20 |
| C₆⁺-KW | 15 - 25 |
| C₅-Diene | 0-0,5 |

Die bevorzugte Zusammensetzung einer partiell hydrierten C₅-Fraktion ist die folgende, wobei die Summe der Gew.-% 100 ergibt.

| Komponente (Kohlenwasserstoffe = KW) | Gehalt (Gew.-%) |
|---|---|
| C₄-KW | 0 - 4 |
| Leichtsiedende C₅-KW | 35 - 60 |
| 2-Methylbuten-2 | 9 - 20 |
| Cyclopentan | 7 - 24 |
| Cyclopenten | 9 - 27 |
| C₆⁺-KW | 0 - 2 |
| C₅-Diene | 0-0,5 |

Aus EP-A-0 799 881 ist bekannt, eine fraktionierte Destillation von partiell hydriertem Pyrolysebenzin zur Gewinnung von Cyclopentan und/oder Cyclopenten durchzuführen, wobei gegebenenfalls ein Gemisch von n-Pentan und Isopentan gewonnen werden kann, indem man in einer Kolonne ein an Cyclopentan und Cyclopenten angereichertes Gemisch als Seitenabzug entnimmt, den restlichen C₅-Schnitt als Kopfprodukt gewinnt und Kohlenwasserstoffe mit 6 und mehr C-Atomen als Sumpfprodukt erhält. Im Rahmen des in EP-A-0 799 881 beschriebenen Verfahrens können ungesättigte Kohlenwasserstoffe einer katalytischen Hydrierung unterworfen werden. Der Nachteil dieses Verfahrens besteht darin, daß ein Seitenabzug aus einer ersten Kolonne, in die ein Vielkomponentengemisch eingespeist wird, immer noch ein Gemisch aus vielen Komponenten darstellt, aus dem sich reine Komponenten nur durch zusätzlichen Aufwand oder nur in ungenügender Reinheit gewinnen lassen. Ein weiterer Nachteil des in EP-A-0 799 881 beschriebenen Verfahrens besteht darin, daß das gewünschte Isopentan lediglich vergesellschaftet mit dem weniger gewünschten n-Pentan gewonnen werden kann. Ein weiterer Nachteil dieses Verfahrens besteht darin, daß sich der Ort des Seitenabzugs mit schwankender Zusammensetzung des eingesetzten partiell hydrierten Pyrolysebenzins infolge des sich damit ändernden Temperaturprofils der Kolonne ebenfalls ändert, so daß eine optimale Gewinnung von Cyclopentan und/oder Cyclopenten nicht möglich ist.

Die DE-A-2256384 offenbart ein Verfahren zur Gewinnung von hochreinem Cyclopenten durch Destillation und anschließende Behandlung des cyclopentadienhaltigen Sumpfes mit einem makroporösen Kationenaustauscher.

Es wurde nun gefunden, daß es vom energetischen Aufwand und von der Qualität der dabei erhältlichen Wertstoffe, also insgesamt aus wirtschaftlichen Gründen, günstiger ist, separate Destillationsstufen vorzusehen und lediglich in einer letzten Destillationskolonne einen Seitenabzug vorzusehen, obwohl ein Fachmann eine solche Vorgehensweise als zu aufwendig ansehen müßte.

Die Erfindung betrifft ein Verfahren zur Gewinnung von Cyclopentan durch destillative Auftrennung eines partiell hydrierten Benzol-Vorlaufs oder einer partiell hydrierten C₅-Fraktion, das dadurch gekennzeichnet ist, daß man
a) in einer ersten fraktionierten Destillation des partiell hydrierten Benzol-Vorlaufs oder der partiell hydrierten C₅-Fraktion als erstes Kopfprodukt Leichtsieder mit Siedepunkten unter dem von Cyclopenten und als erstes Sumpfprodukt Schwersieder mit Cyclopenten und höher als Cyclopenten siedenden Kohlenwasserstoffen entnimmt und entweder
b1) das erste Sumpfprodukt einer zweiten fraktionierten Destillation zuführt und dort Cyclopenten als zweites Kopfprodukt und Cyclopentan sowie höher als Cyclopentan siedende Kohlenwasserstoffe als zweites Sumpfprodukt entnimmt und aus diesem zweiten Sumpfprodukt in einer dritten fraktionierten Destillation Cyclopentan als drittes Kopfprodukt gewinnt und höher als Cyclopentan siedende Kohlenwasserstoffe als drittes Sumpfprodukt abtrennt oder
b2) das erste Sumpfprodukt einer katalytischen Hydrierung unterzieht und anschließend in einer fraktionierten Destillation in Cyclopentan als Kopfprodukt und höher als Cyclopentan siedende Kohlenwasserstoffe als Sumpfprodukt auftrennt oder
b3) aus dem ersten Sumpfprodukt in einer fraktionierten Destillation die höher als Cyclopentan siedenden Kohlenwasserstoffe als Sumpfprodukt abtrennt und das Kopfprodukt einer katalytischen Hydrierung unter Gewinnung von Cyclopentan unterwirft.

Die typischen Zusammensetzungen des partiell hydrierten Benzol-Verlaufs bzw. der partiell hydrierten C₅-Fraktion entsprechen den zuvor bereits aufgeführten.

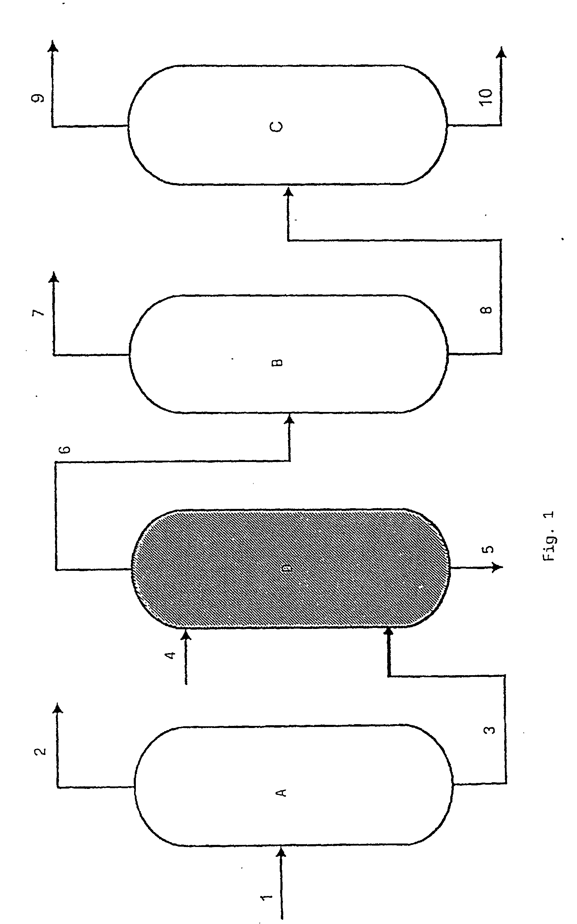

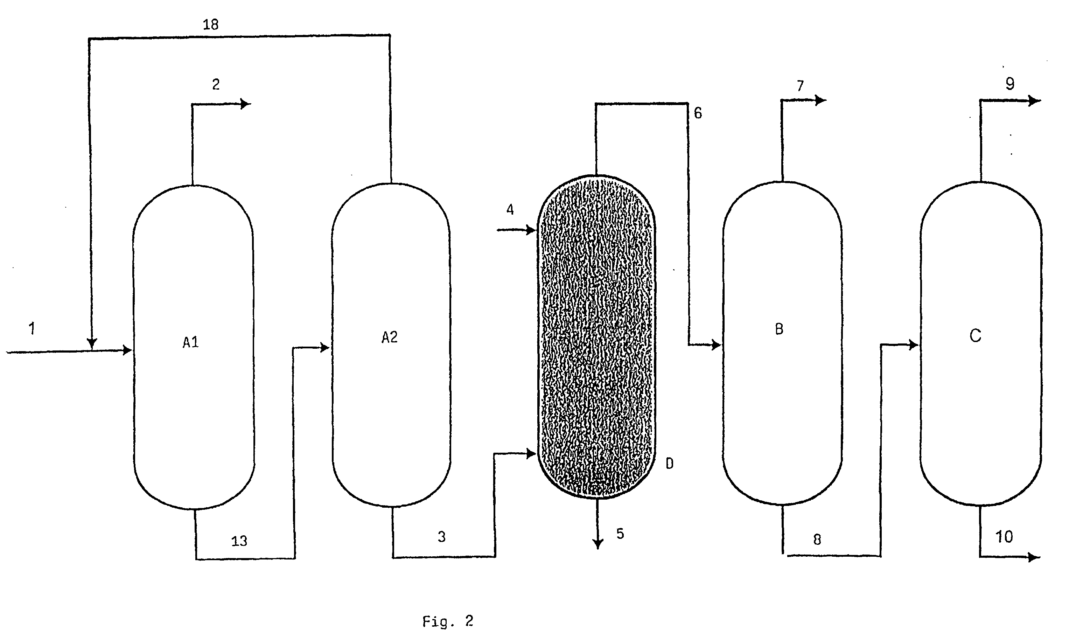

Im Rahmen des erfindungsgemäßen Verfahrens wird gemäß Schritt a) in einer ersten fraktionierten Destillation eine Abtrennung von Leichtsiedern mit Siedepunkten unter dem von Cyclopenten als erstes Kopfprodukt vorgenommen. Diese erste fraktionierte Destillation kann beispielsweise in einer Stufe vorgenommen werden, wobei eine Kolonne mit ausreichender Trennwirkung zur möglichst vollständigen Entfernung der Leichtsieder vorgesehen wird. Es ist jedoch ebenfalls möglich, die erste fraktionierte Destillation zweistufig durchzuführen, wobei dann im Sumpf der ersten Stufe eine erhöhte Konzentration an Leichtsiedern zugelassen werden kann und diese Leichtsieder in der zweiten Stufe vollständig ausdestilliert werden. Die Leichtsieder der zweiten Stufe können hierbei Höhersieder enthalten und werden vorteilhaft auf den Eingang der ersten Stufe zurückgeführt (Fig. 2).

Je nachdem, ob man die erste fraktionierte Destillation gemäß Schritt a) ein- oder zweistufig durchgeführt hat, wird das Sumpfprodukt der ersten bzw. zweiten Stufe einer weiteren fraktionierten Destillation nach b1) zugeführt oder gemäß b2) bzw. b3) weiterbehandelt. Wird das Sumpfprodukt aus Schritt a) gemäß Ausführungsform b1) der zweiten fraktionierten Destillation zugeführt, so erhält man Cyclopenten als zweites Kopfprodukt in hoher Reinheit und Cyclopentan und höher als Cyclopentan siedende Kohlenwasserstoffe als zweites Sumpfprodukt. Das zweite Sumpfprodukt wird abschließend in einer dritten fraktionierten Destillation in Cyclopentan als drittes Kopfprodukt und höher als Cyclopentan siedende Kohlenwasserstoffe mit 6 und mehr C-Atomen als drittes Sumpfprodukt aufgetrennt.

In der Variante nach b2) wird das Sumpfprodukt der ersten fraktionierten Destillation (in einstufiger oder zweistufiger Ausführung) einer katalytischen Hydrierung zur Umwandlung von Olefinen in gesättigte Kohlenwasserstoffe zugeführt. Insbesondere wird hierbei Cyclopenten in das als Treibgas gewünschte Cyclopentan umgewandelt und so die Ausbeute an letzterem erhöht. Im Anschluß an die katalytische Hydrierung erfolgt dann unter Auslassung der zweiten fraktionierten Destillation die Auftrennung in Cyclopentan als Kopfprodukt und die höher als Cyclopenten siedenden Kohlenwasserstoffe als Sumpfprodukt.

Diese Reihenfolge der Variante b2), zunächst katalytisch zu hydrieren und anschließend fraktioniert zu destillieren, kann gemäß Variante b3) auch vertauscht werden. In diesem Fall werden aus dem ersten Sumpfprodukt aus Schritt a) zuerst in einer fraktionierten Destillation die höher als Cyclopentan siedenden Kohlenwasserstoffe als Sumpfprodukt abgetrennt und dann das Kopfprodukt einer katalytischen Hydrierung unter Gewinnung von Cyclopentan aus Cyclopenten unterzogen.

Die Variante b2) erlaubt ferner neben der Gewinnung von Cyclopentan die zusätzliche Gewinnung von Isopentan.

Gegenstand der Erfindung ist somit ferner ein Verfahren zur Gewinnung von Cyclopentan und zusätzlich Isopentan durch destillative Auftrennung eines partiell hydrierten Benzol-Vorlaufs oder einer partiell hydrierten C₅-Fraktion welches dadurch gekennzeichnet ist, daß man
a) in einer ersten fraktionierten Destillation des partiell hydrierten Benzol-Verlaufs oder der partiell hydrierten C₅-Fraktion als erstes Kopfprodukt Leichtsieder mit Siedepunkten unter dem des 2-Methyl-buten-(2) und als erstes Sumpfprodukt Schwersieder mit 2-Methyl-buten-(2) und höher als 2-Methyl-buten-(2) siedenden Kohlenwasserstoffen entnimmt und
b2) das erste Sumpfprodukt einer katalytischen Hydrierung unterzieht und anschließend in einer fraktionierten Destillation in Isopentan als Kopfprodukt, Cyclopentan als Seitenstrom und Kohlenwasserstoffe mit 6 und mehr C-Atomen als Sumpfprodukt auftrennt.

Das Isopentan fällt hierbei als Reinprodukt an und ist insbesondere nicht mit n-Pentan vergesellschaftet, was den unmittelbaren Einsatz in Alkylierungsprozessen oder eine andere Verwendung zuläßt.

Die zusätzliche Gewinnung von Isopentan ist auch über die Variante b3) möglich.

Gegenstand der Erfindung ist daher auch ein Verfahren zur Gewinnung von Cyclopentan und zusätzlich Isopentan durch destillative Auftrennung eines partiell hydrierten Benzol-Vorlaufs oder einer partiell hydrierten C₅-Fraktion, dadurch gekennzeichnet, daß man
a) in einer ersten fraktionierten Destillation des partiell hydrierten Benzol-Verlaufs oder der partiell hydrierten C₅-Fraktion als erstes Kopfprodukt Leichtsieder mit Siedepunkten unter dem des 2-Methyl-buten-(2) und als erstes Sumpfprodukt Schwersieder mit 2-Methyl-buten-(2) und höher als 2-Methyl-buten-(2) siedenden Kohlenwasserstoffen entnimmt und
b3) das erste Sumpfprodukt fraktioniert destilliert, wobei die Höhersieder in Form von Kohlenwasserstoffen mit 6 und mehr C-Atomen als Sumpfprodukt abgetrennt werden und das im wesentlichen Cyclopenten, Cyclopentan und 2-Methyl-buten-2 enthaltende Kopfprodukt zunächst unter Bildung von Isopentan und Cyclopentan katalytisch hydriert und abschließend durch fraktionierte Destillation in Isopentan als Kopfprodukt, Cyclopentan als Seitenstrom und Kohlenwasserstoffe mit 6 und mehr C-Atomen als Sumpfprodukt auftrennt.

Die Höhersieder fallen bei diesem Verfahren jedoch nur noch im Spurenbereich an.

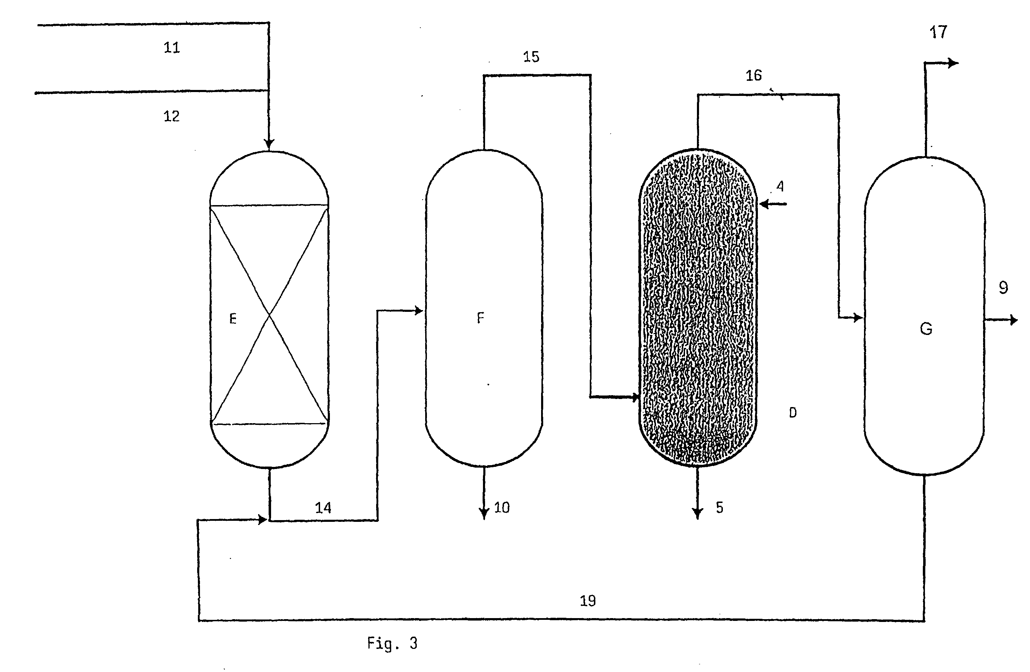

Destillationssümpfe, die bei den verschiedenen fraktionierten Destillationen im Rahmen des erfindungsgemäßen Verfahrens zugunsten der Herstellung reiner Kopfprodukte nicht vollständig ausdestilliert worden sind, können an geeigneter Stelle in das erfindungsgemäße Verfahren zurückgeführt werden, wie dies in Fig. 3 beispielhaft gezeigt wird. Die letztendlich nach Durchführung des erfindungsgemäßen Verfahrens auszuschleusenden Destillationssümpfe mit Kohlenwasserstoffen mit 6 oder mehr C-Atomen können im Verbund einer petrochemischen Anlage in geeigneter Weise, beispielsweise als Rückführstrom zu einem Cracker, eingesetzt werden.

In einer weiteren bevorzugten Ausführungsform des erfindungsgemäßen Verfahrens kann an geeigneter Stelle eine Entfernung von sauerstoffhaltigen organischen Verbindungen durchgeführt werden, indem man das Gemisch der fraktioniert aufzutrennenden Kohlenwasserstoffe einer Wäsche mit Wasser unterwirft. Die sauerstoffhaltigen organischen Verbindungen befinden sich dann in dem als Sumpfprodukt aus einer solchen Wäsche ablaufenden Abwasser, das einer biologischen Reinigung oder einer anderen Entsorgung unterworfen wird. In bevorzugter Form wird diese Wäsche mit Wasser unmittelbar vor der Feindestillation zu den gewünschten Produkten angeordnet.

Bei Durchführung des erfindungsgemäßen Verfahrens gemäß Variante a) und b1) findet die Wäsche mit Wasser bevorzugt zwischen der ersten und zweiten fraktionierten Destillation statt.

Erfolgt das erfindungsgemäße Verfahren in der Variante a) und b2) oder a) und b3), so wird die Wäsche mit Wasser bevorzugt vor oder nach der katalytischen Hydrierung vorgenommen.

Werden die Varianten a) und b2) bzw. a) und b3) unter zusätzlicher Gewinnung von Isopentan durchgeführt, so hat es sich besonders bewährt, die Wäsche mit Wasser unmittelbar vor der letzten fraktionierten Destillation vorzunehmen.

Das erfindungsgemäße Verfahren ist besonders für den Einsatz von partiell hydrierten Benzol-Vorläufen geeignet, die die obengenannte Zusammensetzung haben. Es ist aber durch die separat durchgeführten fraktionierten Destillationsschritte gemäß obiger Beschreibung auch für Benzol-Vorläufe geeignet, die durch einen von der obigen Darstellung abweichenden Anteil an Leichtsiedern und/oder Hochsiedern gekennzeichnet sind. Dies macht es beispielsweise möglich, auch Zukäufe an Benzol-Vorläufen, die nicht der eigenen petrochemischen Anlage entstammen und die daher stark abweichende Zusammensetzungen haben, einzusetzen.

Im erfindungsgemäßen Verfahren kommen für die fraktionierten Destillationen Kolonnen in Frage, wie sie fachmännisch bekannt sind. Sie können demzufolge Destillationsböden der verschiedenen bekannten Ausführungsarten, Füllkörper der verschiedenen bekannten Ausführungsarten oder strukturierte Packungen der verschiedenen bekannten Ausführungsarten enthalten. Die Anpassung solcher Kolonnen an die Anforderungen der verschiedenen fraktionierten Destillationsschritte des erfindungsgemäßen Verfahrens ist prinzipiell bekannt und bedarf keiner weiteren Erläuterung. In einer besonderen Ausführungsart kann die Variante des erfindungsgemäßen Verfahrens mit zusätzlicher Gewinnung von Isopentan in ihrem letzten Verfahrensschritt mit der Seitenstromabnahme des Cyclopentans in einer Trennblechkolonne ausgeführt werden.

Die Durchführung des erfindungsgemäßen Verfahrens ist grundsätzlich unabhängig vom angelegten Druck. Da jedoch die im erfindungsgemäßen Verfahren gewonnenen Stoffe durch ihre niedrigen Siedepunkte bei atmosphärischem Druck gekennzeichnet sind, was insbesondere auf die jeweiligen Kopfprodukte zutrifft, wird man bestrebt sein, zur Kondensation der Kopfprodukte möglichst nicht von teurer Kühlsole und auch möglichst nicht von ebenfalls teurem Kaltwasser Gebrauch zu machen, sondern zur Kondensation möglichst weitgehend normales Kühlwasser einzusetzen. Hierzu wird zur Erhöhung der Kondensationstemperaturen von erhöhtem Druck Gebrauch gemacht. Dieser erhöhte Druck bewegt sich etwa im Bereich von 1,5 bis 10 bar absolut.

Die in den Verfahrensvarianten b2) und b3) vorgesehene katalytische Hydrierung wird unter grundsätzlich bekannten Bedingungen durchgeführt. Als Hydrierkatalysatoren werden auf Trägern angeordnete Edelmetalle, etwa Palladium,. Platin, Ruthenium oder Rhodium, oder Nickel, mit oder ohne bekannte Promotoren eingesetzt. Als Katalysatorträger kommen Al₂O₃, SiO₂, Aktivkohle oder andere für diese Zwecke geeignete Trägermaterialien in Frage. Als Temperatur für die Hydrierung kommt ein Bereich von 20 bis 200°C, bevorzugt 20 bis 60°C, bei Platinmetallen, sowie 120 bis 180°C bei Nickel in Frage. Niedrige Temperaturen in den genannten Bereichen sind bevorzugt, weil dann der Ablauf aus der Hydrierung nicht aufwendig gekühlt werden muß. Der H₂-Druck in der katalytischen Hydrierung liegt im Bereich von 5 bis 100 bar, bevorzugt 5 bis 30 bar. Der eingesetzte Wasserstoff kann sowohl reiner Wasserstoff sein als auch sogenanntes Restgas, das in petrochemischen Anlagen zur Verfügung steht und in vielen Fällen aus etwa 70 % H₂, 25 % CH₄ und geringen Mengen an weiteren hydrierinerten Bestandteilen besteht.

Von den zahlreichen Ausführungsformen des erfindungsgemäßen Verfahrens sind einige anhand der Figuren 1, 2, 3 und 4 beispielhaft dargestellt. In diesen Figuren sind zugunsten einer größeren Übersichtlichkeit Pumpen, Wärmetauscher, Kondensatoren, Heiz- und Druckhalteeinrichtungen nicht aufgeführt. Gleiche Symbole bedeuten gleiche Apparate oder gleiche Stoffströme.

Fig. 1 zeigt drei Destillationskolonnen A, B und C für die erste, zweite und dritte fraktionierte Destillation gemäß Ausführungsform a) und b1). D ist eine Kolonne zur Durchführung der bevorzugt angewandten Wäsche mit Wasser.

Die Stoffströme in Fig. 1 sind folgende:
- 1 =: partiell hydrierter Benzol-Vorlauf oder partiell hydrierte C₅-Fraktion,
- 2 =: erstes Kopfprodukt aus Leichtsiedern mit Siedepunkten unter dem von Cyclopenten,
- 3 =: erstes Sumpfprodukt aus Schwersiedern mit Cyclopenten sowie höher als Cyclopenten siedenden Komponenten,
- 4 =: Wasser zur Wäsche,
- 5 =: Abwasser aus der Wäsche mit Wasser,
- 6 =: mit Wasser gewaschenes erstes Sumpfprodukt von A,
- 7 =: Cyclopenten als zweites Kopfprodukt,
- 8 =: zweites Sumpfprodukt,
- 9 =: Cyclopentan und
- 10 =: drittes Sumpfprodukt mit Kohlenwasserstoffen mit 6 und mehr C-Atomen.

Fig. 2 zeigt eine Variante, bei der die erste fraktionierte Destillation zweistufig. durchgeführt wird. Aus diesem Grund sind anstelle der ersten Destillationskolonne A zwei Destillationskolonnen A1 und A2 vorgesehen, die auch als verschiedene Schüsse einer Kolonne betrieben werden können. Die beiden Kolonnen sind so miteinander verbunden, daß ein Sumpfprodukt 13 aus A1 in A2 eingeführt wird und das Kopfprodukt von A2 als Rückführstrom 18 an den Eingang von A1 zurückgeführt wird. Alle übrigen Stoffströme haben die im Zusammenhang mit Fig. 1 erläuterte Bedeutung.

In Fig. 3 wird eine Ausführungsform aufgezeigt, in der eine katalytische Hydrierung gemäß b2) enthalten ist und die die zusätzliche Gewinnung von Isopentan in reiner Form zuläßt. Auch hier ist eine Wäsche mit Wasser in der Kolonne D vorgesehen. Ansonsten enthält Fig. 3 den Hydrierreaktor E und zwei Destillationskolonnen F und G. In E wird das erste Sumpfprodukt 12 der ersten fraktionierten Destillation; das jedoch zusätzlich zu den in den Strömen 3 und 13 aus Fig. 1 und 2 enthaltenen Stoffen als weiteren Stoff 2-Methyl-buten-(2) enthält, hydriert. Zusätzlich wird H₂ als Strom 11 in E eingeführt. Der Ablauf 14 von E enthält in dieser Ausführungsform im wesentlichen nur noch gesättigte Kohlenwasserstoffe und wird in F eingeführt. F übernimmt in dieser Ausführungsform bezüglich der Ausschleusung von Kohlenwasserstoffen mit 6 und mehr C-Atomen die Funktion von C in Fig. 1 und 2. Das Kopfprodukt 15 von F wird einer Wasserwäsche in D zugeführt. Das Kopfprodukt 16 von D enthält Isopentan, Cyclopentan und geringe Mengen nicht scharf ausdestillierter Kohlenwasserstoffe mit 6 und mehr C-Atomen und wird der Destillationskolonne G zugeführt. Als Seitenabzug von G wird Cyclopentan 9 und als Kopfprodukt von G wird Isopentan 17 entnommen.

Der Sumpfstrom 19 von G enthält die Kohlenwasserstoffe mit 6 und mehr C-Atomen und wird mit Strom 14 vereinigt.

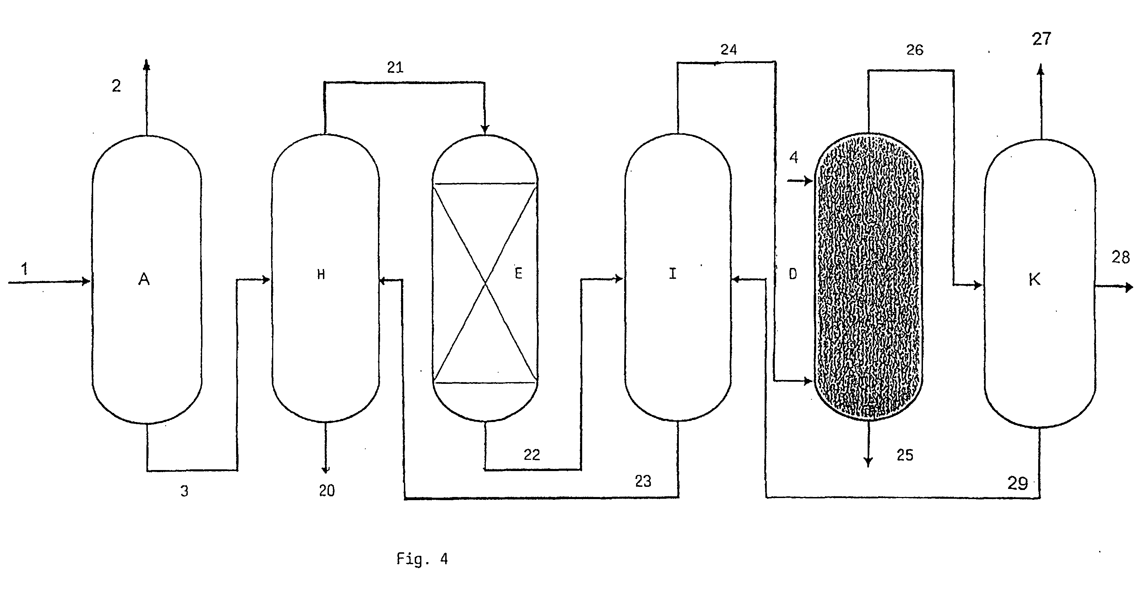

In Fig. 4 wird eine Ausführungsform aufgezeigt, bei der zunächst die erste fraktionierte Destillation gemäß Schritt a) einstufig durchgeführt und'das erste Sumpfprodukt 3 anschließend gemäß Variante b3) einer weiteren fraktionierten Destillation in Destillationskolonne H unterzogen wird, wobei man die Höhersieder 20 als Sumpfprodukt abtrennt. Das Kopfprodukt 21 enthält überwiegend Cyclopenten und Cyclopentan und wird in Reaktor E katalytisch hydriert, wobei als Ablauf 22 eine rohe Cyclopentanfraktion gewonnen wird. Diese wird einer weiteren fraktionierten Destillation in Destillationskolonne I unterzogen. Die Höhersieder dieser fraktionierten Destillation 23 werden in die fraktionierte Destillation H rezirkuliert, während das Kopfprodukt 24 in Kolonne D einer Wäsche mit Wasser zugeführt wird. Das Kopfprodukt 26 enthält im wesentlichen nur noch Cyclopentan und wird in Destillationskolonne K einer letzten Feindestillation unterworfen, wobei das reine Cyclopentan als Seitenstrom 28 abgenommen wird.

### Beispiel

Von 100 Teilen Benzol-Vorlauf werden in einer ersten Destillationskolonne mit etwa 20 theoretischen Trennstufen bei einem Rücklauf von 750 Teilen typischerweise etwa 58 Teile Kopfprodukt und 42 Teile Sumpfprodukt erhalten, dergestalt, daß das Kopfprodukt weniger als 4 Teile cyclische C₅-Kohlenwasserstoffe (Cyclopentan und Cyclopenten) enthält. Das dabei anfallende Sumpfprodukt besteht aus etwa 20 Teilen der cyclischen C₅-Kohlenwasserstoffe, etwa einem Teil 2-Methyl-buten-(2), begleitet von Spuren von Pentenen und etwa 21 Teilen Kohlenwasserstoffen mit 6 und mehr. C-Atomen. Das so erhaltene Sumpfprodukt wird ohne weitere Reinigung in einen Hydrierreaktor gefahren, der unter Zuführung von Wasserstoff an einem handelsüblichen Hydrierkatalysator auf Edelmetall- oder Nickelbasis die ungesättigten Verbindungen in gesättigte Verbindungen überführt. In einer daran anschließenden zweiten Kolonne mit etwa 50 theoretischen Trennstufen werden die 42 Teile des hydrierten Sumpfprodukts bei einem Rücklauf von etwa 80 Teilen weiter destillativ aufgetrennt. Man erhält etwa 22 Teile zweites Kopfprodukt und etwa 20 Teile zweites Sumpfprodukt. Das zweite Kopfprodukt enthält etwa 19 Teile Cyclopentan im Gemisch mit etwa 2 Teilen Isopentan (aus der Hydrierung von 2-Methyl-buten-(2)) sowie etwa 1 Teil höhere Kohlenwasserstoffe. Im Sumpfprodukt gehen nur geringe Mengen (etwa 1 Teil) Cyclopentan verloren. Das Sumpfprodukt besteht somit fast ausschließlich aus Kohlenwasserstoffen mit 6 und mehr C-Atomen und kann beispielsweise als Benzinkomponente oder als Petrolether (Lösungsmittel) eingesetzt werden. Das zweite Kopfprodukt wird anschließend durch eine Wäsche mit Wasser von geringen Mengen darin enthaltener sauerstoffhaltiger polarer Verunreinigungen befreit. Abschließend findet in einer Feinreinigungskolonne die destillative Aufkonzentration des Cyclopentans bis zu einer Reinheit von typischerweise 95 bis 99 Gew.-% statt. Diese letzte Destillationskolonne mit etwa 70 theoretischen Stufen trennt bei einem Rücklauf von etwa 50 Teilen die eingesetzten 22 Teile des Kopfprodukts aus der H₂O-Wäsche in 2 Teile Kopfprodukt (Isopentan), 18 Teile Cyclopentan der angegebenen Reinheit (Seitenstrom) und etwa 2 Teile Sumpfprodukt, bestehend aus nur noch geringen Mengen Cyclopentan und hauptsächlich KohlenWasserstoffen mit 6 und mehr C-Atomen, auf. Das zuletzt genannte Sumpfprodukt wird zur Ausbeutesteigerung in den Ablauf des Hydrierreaktors zurückgeführt.

## Patentansprüche

1. Verfahren zur Gewinnung von Cyclopentan durch destillative Auftrennung eines partiell hydrierten Benzol-Vorlaufs oder einer partiell hydrierten C₅-Fraktion **dadurch gekennzeichnet, daß** man
a) in einer ersten fraktionierten Destillation des partiell hydrierten Benzol-Vorlaufs oder der partiell hydrierten C₅-Fraktion als erstes Kopfprodukt Leichtsieder mit Siedepunkten unter dem von Cyclopenten und als erstes Sumpfprodukt Schwersieder mit Cyclopenten und höher als Cyclopenten siedenden Kohlenwasserstoffe entnimmt und entweder
b1) das erste Sumpfprodukt einer zweiten fraktionierten Destillation zuführt und dort Cyclopenten als zweites Kopfprodukt und Cyclopentan sowie höher als Cyclopentan siedende Kohlenwasserstoffe als zweites Sumpfprodukt entnimmt und aus diesem zweiten Sumpfprodukt in einer dritten fraktionierten Destillation Cyclopentan als drittes Kopfprodukt gewinnt sowie höher als Cyclopentan siedende Kohlenwasserstoffe als drittes Sumpfprodukt abtrennt oder
b2) das erste Sumpfprodukt einer katalytischen Hydrierung unterzieht und anschließend in einer fraktionierten Destillation in Cyclopentan als Kopfprodukt und höher als Cyclopentan siedende Kohlenwasserstoffe als Sumpfprodukt auftrennt oder
b3) aus dem ersten Sumpfprodukt in einer fraktionierten Destillation die höher als Cyclopentan siedenden Kohlenwasserstoffe als Sumpfprodukt abtrennt und das Kopfprodukt einer katalytischen Hydrierung unter Gewinung von Cyclopentan aus Cyclopenten unterwirft.

2. Verfahren zur Gewinnung von Cyclopentan und zusätzlich Isopentan durch destillative Auftrennung eines partiell hydrierten Benzol-Vorlaufs oder einen partiell hydrierten C₅-Fraktion, **dadurch gekennzeichnet, daß** man
a) in einer ersten fraktionierten Destillation des partiell hydrierten Benzol-Verlaufs oder der partiell hydrierten C₅-Fraktion als erstes Kopfprodukt Leichtsieder mit Siedepunkten unter dem des 2-Methyl-buten-(2) und als erstes Sumpfprodukt Schwersieder mit 2-Methyl-buten-(2) und höher als 2-Methyl-buten-(2) siedenden Kohlenwasserstoffen entnimmt und
b2) das erste Sumpfprodukt einer katalytischen Hydrierung unterzieht und anschließend in einer fraktionierten Destillation in Isopentan als Kopfprodukt, Cyclopentan als Seitenstrom und Kohlenwasserstoffe mit 6 und mehr C-Atomen als Sumpfprodukt auftrennt.

3. Verfahren zur Gewinnung von Cyclopentan und zusätzlich Isopentan durch destillative Auftrennung eines partiell hydrierten Benzol-Vorlaufs oder einer partiell hydrierten C₅-Fraktion, **dadurch gekennzeichnet, daß** man
a) in einer ersten fraktionierten Destillation des partiell hydrierten Benzol-Vorlaufs oder der partiell hydrierten C₅-Fraktion als erstes Kopfprodukt Leichtsieder mit Siedepunkten unter dem des 2-Methylbuten-(2) und als erstes Sumpfprodukt Schwersieder mit 2-Methyl-buten-(2) und höher als 2-Methyl-buten-(2) siedenden Kohlenwasserstoffen entnimmt und
b3) das erste Sumpfprodukt fraktioniert destilliert, wobei die Höhersieder in Form von Kohlenwasserstoffen mit 6 und mehr C-Atomen als Sumpfprodukt abgetrennt werden und das im wesentlichen Cyclopenten, Cyclopentan und 2-Methyl-buten-(2) enthaltende Kopfprodukt zunächst unter Bildung von Isopentan und Cyclopentan katalytisch hydriert und abschließend durch fraktionierte Destillation in Isopentan als Kopfprodukt, Cyclopentan als Seitenstrom und Kohlenwasserstoffe mit 6 und mehr C-Atomen als Sumpfprodukt auftrennt.

4. Verfahren nach Anspruch 1, 2 oder 3 **dadurch gekennzeichnet, daß** man die erste fraktionierte Destillation in 2 Stufen vornimmt, wobei in der 1. Stufe die Leichtsieder als Kopfprodukt entnommen werden, das Sumpfprodukt der 1. Stufe vollständig in die 2. Stufe überführt wird, das Kopfprodukt der 2. Stufean den Eingang der 1. Stufe zurückgeführt wird und das Sumpfprodukt der 2. Stufe gemäß Anspruch 1, 2 oder 3 weiterbehandelt wird.

5. Verfahren nach Anspruch 1, 2 oder 3, wobei der partiell hydrierte Benzol-Verlauf oder die partiell hydrierte C₅-Fraktion folgende Zusammensetzung hat, wobei die Summe der Gew.-% 100 ergibt.
| Komponente (Kohlenwasserstoffe = KW) | Gehalt (Gew.-%) |
|---|---|
| C₄-KW | 0-4 |
| Leichtsiedende C₅-KW | 30 - 60 |
| 2-Methylbuten-2 | 8 - 20 |
| Cyclopentan | 6 - 24 |
| Cyclopenten | 8 - 27 |
| C₆⁺-KW | 0 - 25 |
| C₅-Diene | 0 - 0,5 |

## Claims

1. A process for obtaining cyclopentane by the separation by distillation of a partially hydrogenated benzene light end or a partially hydrogenated C₅ fraction, **characterized by** the steps of:
a) withdrawing, in a first fractionated distillation of the partially hydrogenated benzene light end or the hydrogenated C₅ fraction, low boilers having boiling points below that of cyclopentene as a first head product, and high boilers with cyclopentene and hydrocarbons boiling higher than cyclopentene as a first bottoms product; and either
b1) transferring said first bottoms product to a second fractionated distillation where cyclopentene is withdrawn as a second head product and cyclopentane and hydrocarbons boiling higher than cyclopentane are withdrawn as a second bottoms product, and in a third fractionated distillation, obtaining cyclopentane as a third head product from said second bottoms product and separating hydrocarbons boiling higher than cyclopentane as a third bottoms product; or
b2) subjecting said first bottoms product to catalytic hydrogenation, followed by separating it in a fractionated distillation into cyclopentane as a head product and hydrocarbons boiling higher than cyclopentane as a bottoms product; or
b3) separating the hydrocarbons boiling higher than cyclopentane as a bottoms product from said first bottoms product in a fractionated distillation and subjecting the head product to catalytic hydrogenation to obtain cyclopentane from cyclopentene.

2. A process for obtaining cyclopentane and additionally isopentane by the separation by distillation of a partially hydrogenated benzene light end or a partially hydrogenated C₅ fraction, **characterized by** the steps of:
a) withdrawing, in a first fractionated distillation of the partially hydrogenated benzene light end or the partially hydrogenated C₅ fraction, low boilers having boiling points below that of 2-methylbutene-(2) as a first head product, and high boilers with 2-methylbutene-(2) and hydrocarbons boiling higher than 2-methylbutene-(2) as a first bottoms product; and
b2) subjecting said first bottoms product to catalytic hydrogenation, followed by separating it in a fractionated distillation into isopentane as a head product, cyclopentane as a side stream and hydrocarbons having 6 and more carbon atoms as a bottoms product.

3. A process for obtaining cyclopentane and additionally isopentane by the separation by distillation of a partially hydrogenated benzene light end or a partially hydrogenated C₅ fraction, **characterized by** the steps of:
a) withdrawing, in a first fractionated distillation of the partially hydrogenated benzene light end or the partially hydrogenated C₅ fraction, low boilers having boiling points below that of 2-methylbutene-(2) as a first head product, and high boilers with 2-methylbutene-(2) and hydrocarbons boiling higher than 2-methylbutene-(2) as a first bottoms product; and
b3) subjecting the first bottoms product to fractionated distillation to separate off high boilers in the form of hydrocarbons having 6 and more carbon atoms as a bottoms product, and subjecting the head product, which substantially contains cyclopentene, cyclopentane and 2-methylbutene-(2), first to catalytic hydrogenation to form isopentane and cyclopentane, followed by separation by fractionated distillation into isopentane as a head product, cyclopentane as a side stream and hydrocarbons having 6 and more carbon atoms as a bottoms product.

4. The process according to claim 1, 2 or 3, **characterized in that** said first fractionated distillation is performed in two stages, wherein, in the first stage, the low boilers are withdrawn as a head product, the bottoms product of the first stage is completely transferred to the second stage, the head product of the second stage is recirculated to the feed of the first stage, and the bottoms product of the second stage is further treated according to claim 1, 2 or 3.

5. The process according to claim 1, 2 or 3, wherein said partially hydrogenated benzene light end or partially hydrogenated C₅ fraction has the following composition, wherein the sum of the weight percents amounts to 100:
| component | content (% by weight) |
|---|---|
| C₄ hydrocarbons | 0 to 4 |
| low-boiling C₅ hydrocarbons | 30 to 60 |
| 2-methylbutene-2 | 8 to 20 |
| cyclopentane | 6 to 24 |
| cyclopentene | 8 to 27 |
| C₆+ hydrocarbons | 0 to 25 |
| C₅ dienes | 0 to 0.5 |

## Revendications

1. Procédé pour la récupération de cyclopentane par séparation distillatrice d'une fraction de tête de la distillation de benzène partiellement hydrogénée ou d'une fraction en C₅ partiellement hydrogénée, **caractérisé en ce que**
a) dans une première distillation fractionnée de la fraction de tête de distillation de benzène partiellement hydrogénée ou de la fraction en C₅ partiellement hydrogénée, en tant que premier produit de tête, on prélève la fraction à bas point d'ébullition avec des points d'ébullition au-dessous de celui des cyclopentènes et en tant que premier produit de bas de colonne, on prélève la fraction à haut point d'ébullition avec des cyclopentènes et des hydrocarbures à point d'ébullition supérieur aux cyclopentènes et, ou bien
b1) on amène le premier produit de bas de colonne à une seconde distillation fractionnée et on y prélève des cyclopentènes en tant que second produit de tête et du cyclopentane ainsi que des hydrocarbures à point d'ébullition supérieur au cyclopentane en tant que second produit de bas de colonne et à partir de ce second produit de bas de colonne, dans une troisième distillation fractionnée, on récupère du cyclopentane en tant que troisième produit de tête et des hydrocarbures à point d'ébullition supérieur au cyclopentane en tant que troisième produit de bas de colonne ou bien
b2) le premier produit de bas de colonne est soumis à une hydrogénation catalytique et consécutivement, dans une distillation fractionnée, il est séparé en cyclopentane comme produit de tête et en hydrocarbures à point d'ébullition supérieur au cyclopentane en tant que produit de bas de colonne ou bien
b3) dans une distillation fractionnée, on sépare à partir du premier produit de bas de colonne les hydrocarbures à point d'ébullition supérieur au cyclopentane en tant que produit de bas de colonne et le produit de tête est soumis à une hydrogénation catalytique, en récupérant du cyclopentane à partir de cyclopentènes.

2. Procédé pour la récupération du cyclopentane et à titre supplémentaire d'isopentane par séparation distillatrice d'une fraction de tête de benzène partiellement hydrogénée ou d'une fraction en C₅ partiellement hydrogénée, **caractérisé en ce que**
a) dans une première distillation fractionnée de la fraction de tête de benzène partiellement hydrogénée ou de la fraction en C₅ partiellement hydrogénée, on prélève en tant que premier produit de tête la fraction à bas point d'ébullition avec des points d'ébullition au-dessous de celui du 2-méthyl-butène-(2) et en tant que premier produit de bas de colonne la fraction à haut point d'ébullition avec le 2-méthyl-butène-(2) et des hydrocarbures à point d'ébullition supérieur à celui du 2-méthyl-butène-(2) et
b2) on soumet le premier produit de bas de colonne à une hydrogénation catalytique et consécutivement, dans une distillation fractionnée, on sépare dans l'isopentane en tant que produit de tête, le cyclopentane en tant que courant latéral et les hydrocarbures avec 6 atomes ou plus d'atomes C en tant que produit de bas de colonne.

3. Procédé pour la récupération de cyclopentane et à titre supplémentaire d'isopentane par séparation distillatrice d'une fraction de tête de benzène partiellement hydrogénée ou d'une fraction en C₅ partiellement hydrogénée, **caractérisé en ce que**
a) l'on prélève dans une première distillation fractionnée de la fraction de tête de benzène partiellement hydrogénée ou de la fraction en C₅ partiellement hydrogénée en tant que premier produit de tête des fractions à bas point d'ébullition avec des points d'ébullition au-dessous de celui du 2-méthyl-butène-(2) et en tant que produit de bas de colonne des fractions à haut point d'ébullition avec le 2-méthyl-butène-(2) et des hydrocarbures à point d'ébullition supérieur au 2-méthyl-butène-(2) et
b3) le premier produit de bas de colonne est distillé, fractionné, tandis que les fractions à point d'ébullition supérieur sous forme d'hydrocarbures avec 6 ou plus d'atomes C sont séparés en tant que produit de bas de colonne et le produit de tête contenant essentiellement les cyclopentènes, cyclopentane et 2-méthyl-butène-(2) est d'abord hydrogéné catalytiquement avec formation d'isopentane et de cyclopentane et pour finir, il est séparé par distillation fractionnée dans l'isopentane en tant que produit de tête, de cyclopentane en tant que courant latéral et en hydrocarbures avec 6 et plus d'atomes C en tant que produit de bas de colonne.

4. Procédé selon la revendication 1,2 ou 3 **caractérisé en ce que** l'on procède à la première distillation fractionnée en 2 étapes, tandis que dans la première étape on prélève les fractions à bas point d'ébullition en tant que produit de tête, le produit de bas de colonne de la première étape est complètement transféré dans la seconde étape, le produit de tête de la seconde étape est ramené à l'entrée de la première étape et le produit de bas de colonne de la seconde étape est traité ultérieurement selon la revendication 1, 2 ou 3.

5. Procédé selon la revendication 1,2 ou 3, dans lequel la fraction de tête de benzène partiellement hydrogénée ou la fraction en C₅ partiellement hydrogénée a la composition suivante, la somme des % en poids donnant 100.
| Composants (hydrocarbures = KW) | Teneur (% en poids) |
|---|---|
| KW en C₄ | 0-4 |
| KW en C₅ à bas point d'ébullition | 30-60 |
| 2-méthyl-butène-2 | 8-20 |
| cyclopentane | 6-24 |
| cyclopentène | 8-27 |
| KW en C₆⁺ | 0-25 |
| Diène en C₅ | 0-0,5 |
